(19) Europäisches Patentamt
European Patent Office
Office européen des brevets

(11) Publication number: **0 320 164 B1**

(12) **EUROPEAN PATENT SPECIFICATION**

(45) Date of publication of patent specification: **08.02.95**

(51) Int. Cl.[6]: **C08B 37/08**, A61K 7/48

(21) Application number: **88311301.1**

(22) Date of filing: **29.11.88**

# (54) Method for preparing hylan and novel hylan product.

(30) Priority: **10.12.87 US 130889**

(43) Date of publication of application:
**14.06.89 Bulletin 89/24**

(45) Publication of the grant of the patent:
**08.02.95 Bulletin 95/06**

(84) Designated Contracting States:
**BE CH DE FR GB IT LI NL SE**

(56) References cited:
**GB-A- 2 151 244**
**GB-A- 2 172 295**
**US-A- 4 582 865**
**US-D- 4 141 973**

**BIOCHIM. BIOPHYS. ACTA, vol. 21, 1956, pages 506-518; K. MEYER et al.: "The acid mucopolysaccharides of connective tissue"**

**OJE 11/1989, p. 441**

(73) Proprietor: **Biomatrix, Inc.**
**65 Railroad Avenue**
**Ridgefield**
**N.J. 07657 (US)**

(72) Inventor: **Balazs, Endre A.**
**200 Old Palisade Road**
**Fort Lee, NJ 07024 (US)**
Inventor: **Leshchiner, Adolf**
**623 Prospect Avenue**
**Fairview, NJ 07022 (US)**
Inventor: **Leshchiner, Adelya**
**623 Prospect Avenue**
**Fairview, NJ 07022 (US)**
Inventor: **Larsen, Nancy E.**
**1301 Summit Avenue**
**Southfield NY 10975 (US)**
Inventor: **Band, Philip**
**2765 West 5th Street**
**Brooklyn**
**New York (US)**

(74) Representative: **Allam, Peter Clerk et al**
**LLOYD WISE, TREGEAR & CO.**
**Norman House**
**105-109 Strand**
**London WC2R 0AE (GB)**

**EP 0 320 164 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a new method for obtaining hylan as well as new hylan preparations.

Hyaluronan, sometimes hereinafter also referred to as a hyaluronic acid (HA) is a naturally occurring high molecular weight glycosaminoglycan having a repeating disaccharide unit of D-glucuronic acid and N-acetylglucosamino-2-acetamido-2-desoxy-D-glucose by $\beta 1 \rightarrow 3$ glucosidic bond. The disaccharides are joined to form an unbranched uncrosslinked polysaccharide chain by $\beta 1 \rightarrow 4$ glucosidic bonds.

HA is found in animal tissues such as umbilical cord, vitreous, synovial fluid, rooster and chicken combs, skin, etc. The molecular weight of purified HA has been reported in the literature to be within the range of 50,000 to 8,000,000 depending on the source, method of isolation and method of determination of molecular weight (Balazs, E.A., Fed. Proceed. 17, 1086-1093 (1958)). The most advanced method for HA recovery and purification on an industrial scale is described in U.S. Patent No. 4,141,973 (E.A. Balazs). According to this method, an ultra-pure HA with a protein content of less than 0.5% by weight and a molecular weight more than 1,200,000 is obtained by water extraction from rooster combs or human umbilical cord.

In GB-A-2172295, we have disclosed a chemically modified hyaluronic acid preparation, characterized by novel chemical physiochemical and rheological properties which are described in detail. We gave the name Hylan (hereinafter sometimes referred to as HY) to this chemically modified hyaluronic acid preparation. The method described in GB-A-2172295 for preparing hylan comprises the steps of:

(a) treating animal tissue containing hyaluronic acid with an aqueous treating mixture including a substance reactive with proteins and hyaluronic acid to effect chemical modification of the hyaluronic acid contained in the tissue, in situ,
(b) removing excess treating mixture from the reaction mixture,
(c) extracting the chemically modified hyaluronic acid from the treated animal tissue with water,
(d) separating the extract containing the chemically modified hyaluronic acid from the treated animal tissue, and
(e) recovering the chemically modified hyaluronic acid from the extract.

Among the substances reactive with proteins and hyaluronic acid used to effect in situ chemical modification of the hyaluronic acid contained in the animal tissue disclosed in GB-A-2172295 are aldehydes, for example formaldehyde, glutaraldehyde and glyoxal.

In GB-A-2172295, we showed that the pH of the extracting media strongly affects the yield of hylan in a way such that an increase in pH towards the alkaline condition results in a greater yield but, at the same time, brings the molecular weight down because of hylan degradation. We therefore recommended that the pH be kept within the range of 6 to 8.5 during the extraction step.

We have now found, in accordance with the present invention, that an increased yield of hylan can be achieved in the method of GB-A-2172295 when the pH is at least 9.5 and the temperature during the extraction is kept substantially below ambient (i.e. 20 °C) temperature, i.e. below 16 °C and preferably below 5 °C, which allows one to keep the molecular weight of the hylan at a substantially high level.

More particularly, the present invention provides a method of obtaining a chemically modified hyaluronic acid preparation comprising:

(a) treating animal tissue containing hyaluronic acid with an aqueous treating mixture including an aldehyde to effect chemical modification of the hyaluronic acid contained in the tissue, in situ,
(b) removing excess treating mixture from the reaction mixture,
(c) extracting the chemically modified hyaluronic acid from the treated animal tissue with water at a temperature below 16 °C and an alkaline pH of at least 9.5 for at least 6 hours,
(d) separating the extract containing the chemically modified hyaluronic acid from the treated animal tissue, and
(e) recovering the chemically modified hyaluronic acid from the extract.

The time of the extraction in the method of this invention can vary from 6 hours up to several days depending upon the desired yield and molecular weight of the polymer, and also upon the alkali concentration and the temperature. The pH preferably is in the range from 9.5 to 14 but may be higher.

The alkaline conditions during the extraction can be created by using various alkaline substances including inorganic alkalis such as sodium or potassium hydroxide, ammonium hydroxide, sodium or potassium carbonate, or organic bases such as triethylamine, triethanolamine, etc. We have found that in some cases it may be preferable to use a mixture of alkaline substances, e.g. a mixture of sodium and ammonium hydroxides.

It has also now been found that a sterile, non-inflammatory and essentially pyrogen-free product can be easily obtained by using the process according to the present invention. We have now found that this type

of product is obtained much more readily when washed, whole rooster or hen combs are treated with an alkaline solution for a relatively short time. This extra step is performed after the initial washing of the combs as described in GB-A-2172295. The best results are obtained when the concentration of the inorganic alkali, e.g. sodium hydroxide, is from 0.05M to 4M, preferably from 0.1M to 1M, and most preferably from 0.15 to 0.25M. The time of the treatment depends upon the alkali concentration and can vary from 5 minutes to several hours, preferably from 10 minutes to 1 hour. It should be understood that any type of alkaline substance can be used, including inorganic and organic bases, providing that these substances can create a pH in the treatment solution higher than 12. One can use a solution of an alkali in water or in a mixture of water with a water-miscible solvent such as acetone, ethanol, isopropanol, etc. The treated combs can be washed from alkali with water or with a weak solution of an acid followed with water. The thusly treated combs are then subjected to treatment in the aldehyde-containing mixture according to the procedure described in the parent application.

We have found that the content of the combined aldehyde in hylan products recovered in the method according to the present invention can be substantially less than the 0.005 to 0.05 wt. % which was stated in GB-A-2172295. The lowest level we have been able to detect in some of the samples is around 0.0002 wt. %. Thus, the method of the present invention can lead to the recovery of a chemically modified hyaluronic acid preparation characterized by the presence of from 0.0002 to 0.005% by weight of aldehyde cross-linking groups covalently bonded to the hyaluronic acid polymer chains.

In order to obtain more information pertaining to the chemistry of the hylan formation a second experiment (Example 8) with radio-labelled formaldehyde was carried out which differs in some respects from the experiment described in Example 12 of GB-A-2172295. The results of these two experiments allow one to draw the following conclusions:

1. Small amounts of formaldehyde are strongly, supposedly, covalently bound to hylan macromolecules. This formaldehyde cannot be removed by repeated precipitations, washing with a solvent, drying of dialysis.

2. Substantial amounts of this bound formaldehyde are removable by dialysis after the polysaccharide moiety of the hylan is digested with hyaluronan and chondroitin sulfate degrading hyaluronidases. It is very probable that this formaldehyde is bound to dialyzable peptides or combinations of peptides with oligosaccharides. Some of this formaldehyde can be removed by lyophilization which indicates that it is bound loosely.

3. The strong evidence of the reaction of formaldehyde with the protein moieties in hylan was obtained by autoradiography. It should be especially mentioned, that the most significant incorporation of formaldehyde is observed in the protein band which is always present in hylan samples and is one of the major bands in the electrophoretic patterns of hylan.

As with the method of our earlier invention described in GB-A-2172295, any substance which reacts with proteins in a water-containing medium can be used for the purpose of the present invention. We have found that the most advantageous substance is formaldehyde. Other aldehydes such as glutaraldehyde or glyoxal also can be used in the method according to the present invention.

The treatment of the tissue can be carried out in a water solution of the reagent. However, if this is done, there will be a substantial loss of HY because of its good solubility in water. For this reason, it is preferable to carry out the treatment of the tissue in a mixture of water and a water-miscible organic solvent. The solvent should not react with the reagent used for the protein immobilization. Among these solvents are lower ketones such as acetone or methyl ketone, alcohols such as ethanol or isopropanol, aprotic solvents such as dimethylformamide, dimethylacetamide or dimethylsulfoxide and others. When such a solvent is mixed with a tissue which contains a large amount of water, usually 80-90% or more by weight, a water-solvent mixture is formed. The water-solvent ratio in the mixture can be adjusted to any desired level by changing the solvent/tissue ratio or by adding water to the mixture. The preferable water/solvent ratio is determined by HY solubility in the sense that the HY should not be soluble in the mixture used for the treatment of the tissue. The HY solubility depends upon the type of solvent used, water/solvent ratio, the presence and concentration of any electrolyte in the mixture and the pH of the mixture. The HY solubility can be substantially reduced by introducing an electrolyte into the mixture. Any type of electrolyte can be used which is soluble in the water-solvent mixture and which provides the desired pH of the mixture. For example, when acetone is used as a solvent sodium acetate can be conveniently used as a soluble electrolyte.

The composition of the mixture for treating the tissue can vary over a broad range depending upon the nature of the tissue, the type of solvent used, the type of electrolyte, etc. As mentioned above, HY from the tissue should not be soluble in the treating mixture but the latter should contain enough water to allow the tissue to swell so as to facilitate the reaction between the reagent and the tissue polymers. We have found

that in the case of rooster combs as a source of HY, the composition of the mixture, taking into account the water from the combs, can be in the following range, % by weight: water 10-50, solvent 40-85, electrolyte 0-20, reagent 0.2-10. Several different solvents can be used in the same treating mixture, if desired. We have found it to be advantageous to use small amounts of water-immiscible solvents, such as chloroform, in the treating mixture. The contents of these solvents in the mixture can be from 0.5 to 10% by weight.

The pH of the treating mixture can be varied depending upon the nature of the reagents, composition of the mixture, temperature and time of the treatment. In recovery of HY from animal tissue according to the present invention, the following considerations are very important. Some of the reagents, formaldehyde, for example, can react with hydroxyl groups of HA macromolecules at low pH to give a cross-linked polymer, insoluble in water. A prolonged treatment in a medium with a relatively high pH leads to the degradation of HA and only a low molecular weight polymer can be recovered. We have found that when an aldehyde type of reagent is used according to the present invention the best results are obtained with pH close to neutral, for example, in the range from 4 to 10.

The ratio of the treating mixture to the tissue can vary over broad limits. The lower limit is determined, usually, by the provision that the tissue, which is quite bulky in the case of rooster combs, should at least be fully covered with the mixture. The upper limit can be chosen based on economical consideration. In treatment of rooster combs according to the present invention, the ratio of the treating mixture to the tissue (calculated on dry weight of the tissue) is usually more than 10:1.

The temperature affects the efficiency of the treatment according to the present invention. However, since HY is susceptible to hydrolysis at elevated temperature, it is preferable to carry out the treatment at room temperature or below in order to obtain a high molecular weight product.

The time needed to perform the treatment depends upon many factors including the composition of the mixture, the nature of the tissue, temperature, etc. It is assumed that the limiting factor in the treatment is the diffusion of the reagent into the tissue slices. For this reason, the size of these slices is an important parameter. We have found that in treatment of rooster combs sliced into pieces of 1-3 mm thickness, the time of the treatment can be in the range of 4-24 hours.

The tissue treated as described above is then washed with a solvent or a solvent/water mixture to remove the excess treating mixture from the tissue. It is convenient to use the same solvent as is used in the mixture for the tissue treatment. One can use any number of washings but it has been found that one washing gives satisfactory results.

The washed tissue is then directly extracted with water by the method according to the present invention to recover the chemically modified HA. We have found that the efficiency of the extraction depends not only upon the pH of the extracting media but also upon the water/tissue ratio, temperature and time. We have also found that the efficiency of the extraction of the treated tissue can be substantially increased by first drying the treated tissue to remove the solvent which was used in the treating and washing steps. The best results are obtained when the tissue is dried to 1/4 to 1/2 of its original weight.

The water/tissue ratio in the extraction step is chosen based on several considerations. First of all, there should be enough of the liquid phase to cover the tissue during extraction. On the other hand, the amount of water should not be too large in order to have as high a concentration of HY in the extract as possible so as to reduce the amount of precipitant in the next step of the process. We have generally found that the preferred weight ratio of water to tissue is from 2 to 5 based on the weight of the tissue being treated.

As already noted, we have found in accordance with the present invention that in order to obtain high yields of hylan, the pH of the extracting media should be at least 9.5 and the temperature kept below 16 °C.

The time needed for extracting the maximum amount of HY from the treated tissue varies substantially with other parameters of the extraction, such as pH, liquid/tissue ratio and intensity of stirring. We have found that in the extraction of rooster combs with water, good results can be obtained when the treated combs are extracted for from 6 hours to several days.

The mixture of treated tissue and extracting media can be stirred during the extraction or left without any stirring. Clearly, stirring will increase the diffusion rate of HY molecules from the tissue into the extract. On the other hand, vigorous stirring can lead to degradation of HY and, hence, to a decrease in the molecular weight. In addition, we have found that vigorous stirring causes tissue disintegration which makes it difficult to separate the tissue from the extract. Therefore, it is preferable to carry out the extraction step without or with very slow and gentle stirring.

After extraction, the tissue is separated from the extract using any of several conventional methods including filtration, centrifugation, decantation and the like. We have found that the most simple and economical way is filtration. Depending upon the kind of tissue used as a starting material, it may be preferable to use a two-step filtration. Thus, in the case of rooster combs, large pieces of tissue can be easily separated by filtration through a nylon mesh and the fine purification of the extract can be achieved

by filtration through any dense filter media, e.g. a cellulosic material.

The HY concentration in the extract depends upon many factors including pH during extraction, time, liquid/tissue ratio, and intensity of stirring. Usually it is in the range of from 0.3 to 3.0 mg/ml and, sometimes, even higher. In some cases, when the HY concentration in the extract is on the lower side, we have found it desirable to run a second extraction of the tissue. We have also found that the product precipitated from the second extract usually has a higher molecular weight as compared to the HY from the first extract. This can be explained by the fact that low molecular weight fraction of HY diffuse more easily from the tissue into the extract and the product precipitated from the first extract is enriched with these fractions.

One can use more than two extractions to achieve as high a yield as possible, but the concentration of HY in an extract decreases with each consecutive extraction.

HY can be recovered from the filtrates by any method known in the prior art, for example by precipitation with a water-miscible solvent such as acetone, ethanol, isopropanol, etc, as described in GB-A-2172295. This precipitation can be done in the presence of an acid such as hydrochloric, sulfuric, phosphoric, etc, or in the presence of neutral electrolytes such as sodium acetate, sodium chloride and their salts. HY and its salts are usually precipitated as a white fiber-like material or a powder. The precipitate can be washed with the same solvent which is used for precipitation, or with any other solvent mixture which will not dissolve the product, for example, ether. The washed product can be dried with any conventional means or can be stored under a layer of a solvent such as acetone, ethanol, etc. Alternately, the filtrate can be freeze-dried.

However, in accordance with another new aspect of this invention, the hylan solutions obtained in the filtration step can be directly used without precipitation and redissolving. Because this approach eliminates the precipitation step usually involving large amounts of organic solvents the product obtained is substantially cheaper than normally produced hylan. It is preferable to use the process with alkaline extraction since the concentration of hylan in the filtrate and, hence, the yield is substantially higher as compared to the extraction at neutral pH. The excess alkali in the product can be neutralized with any acid including inorganic acids such as hydrochloric, sulfuric, phosphoric, etc. or organic acids such as acetic, citric, p-aminobenzoic, stearic, etc., or with polymeric acids such as poly(acrylic acid), poly(vinyl sulfonic acid), etc. The choice of acid depends upon the final use of the product. Thus, when the hylan solution is used in cosmetic formulations, the use of poly(acrylic acid) provides for obtaining of gels; the use of stearic acid is convenient in preparing cosmetic emulsions (creams, lotions, etc.), and the use of p-aminobenzoic acid can provide formulations with sunscreening properties. However, the choice of a specific acid does not impose any particular limitations on this aspect of the present invention.

It is also clear that any additional steps known in the prior art and used in HA purification can be included in the process according to the present invention. For example, to remove pyrogens or inflammatory agents, extraction by well known solvents, such as chloroform, in which HY is insoluble but lipoproteins, glycolipids or glycolipoproteins are soluble or separated, can be used.

The process according to the present invention allows one to obtain HY products with properties varying over a broad range. The following properties can be evaluated using the methods cited below.

The HY concentration in solutions is determined by hexuronic acid assay using the automated carbazole method (E.A. Balazs, K.O. Berntsen, J. Karossa and D.A. Swann, Analyt. Biochem. 12, 547-558 (1965)). The hexosamine content is determined by the automated colorimetric method (D.A. Swann and E.A. Balazs, Biochem. Biophys. Act 130, 112-129 (1966)). Protein content of HY solutions is determined by the phenol reagent method (Lowry et al, J. Biol. Chem 193, 265-275 (1951)).

The formaldehyde content in the product is determined by hydrolysis of about 0.1 g of the sample in 10 ml of 10% aqueous sulfuric acid for 2 hours with boiling followed by steam distilling free formaldehyde off the solution obtained and determining formaldehyde in the distillate using a colormetric method with chromotropic acid (M.J. Boyd and M.A. Logan, J. Biol. Chem., 146, 279 (1942)).

The limiting viscosity number (intrinsic viscosity) defined as lim (n/no - 1)/c where n and no are the viscosities of the solution and the solvent respectively, and c the concentration of HY in g/cc. The measurements are carried out in aqueous 0.20 M NaCl solutions in an Ubbelohde capillary type dilution viscosimeter. The viscosity-average molecular weight is calculated by the equation $[n] = 0.0228 M^{0.81}$ - (R.C. Cleland and J.L. Wang, Biopolymers 9, 799 (1970)).

The weight-average molecular weight is determined by the low angle laser light scattering method using a Chromatix KMX-6 instrument equipped with a helium-neon laser set at 632.8 nm. The weight-average molecular weight is also calculated from the sedimentation and diffusion constants determined in an analytical ultra-centrifuge.

The dynamic light scattering method is used to evaluate the aggregation of the molecules in relatively concentrated solutions of HY. This method gives the distribution of equivalent spherical diameters (ESD) in solution.

Rheological properties are evaluated with the Bohlin Rheometer System which is a computerized rheometer which can operate in three modes: viscometry, oscillation and relaxation. The following parameters are measured for the HY solution: viscosity for the broad range of shear rates, dynamic viscosity, dynamic storage moduli and dynamic loss moduli for various oscillation frequencies and relaxation time.

As mentioned above, the product according to the present invention (HY) is a polymer, obtained as a result of an in situ chemical reaction between HA and an aldehyde cross-linking agent, such as formaldehyde. We have found, by chemical analysis of HY, that the content of the combined formaldehyde in products obtained from the rooster combs treated with a formaldehyde-containing mixture, is in the range of from 0.0002 to 0.05 wt. % calculated on the weight of polymer, depending upon the various parameters of the treatment.

The protein content in the product obtained according to the present invention is usually not more than 0.5% as calculated on the weight of a dry polymer and can be as little as 0.1% and even less.

The chemical modification of hyaluronic acid by covalent attachment of a cross-linking agent to its macromolecules, in other words the changes in the primary structure of the polymer, substantially affect its physicochemical parameters such as molecular weight and molecular size, intermolecular interaction, and rheological properties of polymer solutions, as well.

HY obtained according to the present invention can have a very high molecular weight. Thus, the limiting viscosity number can be higher than 7,000 cc/g which corresponds to a viscosity-average molecular weight of around $6 \times 10^6$. The weight-average molecular weight from the light-scattering data can reach a value of $13 \times 10^6$. It should be understood that a polymer with substantially lower molecular weight, e.g., $1 \times 10^6$ or less, can be easily obtained with the method according to the present invention, if desired. Similarly, a polymer with any desired molecular weight can be obtained if HY, at any stage of its recovery and purification, is exposed to agents which are known to break the glucosidic bond of the polysaccharide chain. Such well known agents are specific enzymes, e.g. hyaluronidase, free radical generating systems, shear forces, heat, strong alkalies and acids, etc.

The following parameters characterize the elastic properties of the polymer solutions in the best way: dynamic storage moduli (G′), the frequency of "cross-over point" (a point at which dynamic storage module G′ becomes greater than dynamic loss moduli G″), phase angle and relaxation time.

HY forms very viscous solutions in water or water solution of electrolytes. The viscosity of the solution depends upon the polymer concentration, electrolyte content, temperature and decreases with shear rate, i.e. the HY solutions have substantial pseudoplasticity.

When the rheological properties of HY solutions are considered, one should understand that these properties are greatly dependent upon the molecular weight of the product, as in the case of any other polymer. It was mentioned above that HY can be obtained with a molecular weight varying over a broad range and the rheological properties will vary accordingly. Thus, we have found that for the ultra-high molecular weight product (limiting viscosity number higher than 4500 cc/g) the viscosity of 1 wt. % solution in 0.15 M NaCl solution in water is up to 1000 Pa.s and even higher at shear rate 0.055, whereas it is only about 2 Pa.s for 1 wt. % solution of polymer with limiting viscosity number about 1000 cc/g. We have found that the elastic properties of HY solutions also depend upon the polymer molecular weight. Thus, the dynamic storage module G′ for a 1 wt % solution of ultra-high molecular weight HY in 0.15 M NaCl is about 40 Pa at a frequency 0.01 Hz, whereas it is only about 0.2 Pa for a solution of HY with limiting viscosity number about 1000 cc/g. The frequency of the "cross-over point" which characterizes in a very good way the ratio between elastic and viscous properties of polymer solutions is usually less than 0.025 Hz for 1 wt. % HY solutions in 0.15 M NaCl at 25°C when molecular weight of the polymer is in the range from about $1.5 \times 10^6$ to $8 \times 10^6$ and higher.

The chemically modified hyaluronic acid, HY, prepared according to the present invention can be successfully used for many applications in the biomedical field and in cosmetics, for example, as a tool in viscosurgery, for coatings to improve the biocompatibility of various materials, as a component of various pharmaceutical preparations, in skin care products, etc. The improved properties of this polymer, such as an increased elasticity (see GB-A-2172295), give substantial benefits when HY is used.

HY can also be used as a starting material for new products obtained through additional chemical modification, such as cross-linking with conventional cross-linking agents. We have found that the special properties of chemically modified HA according to the present invention and its solutions provide an opportunity to obtain the above mentioned additionally modified products which also possess some unusual properties. Thus, we have found that a water-insoluble jelly-like material can be obtained from the product

according to the invention by cross-linking with divinyl sulfone in alkaline solution. This material is a highly swollen gel. The concentration of the polymer in the gel depends upon the composition of the liquid phase which can be water or water solutions of various low-molecular weight substances, such as electrolytes. In the case of physiological salt solution (0.15 M NaCl in water), the polymer concentration can be in the range from 0.15 to 0.40 wt. %. This material possesses very interesting rheological properties (see FIGS. 5, 6 and 7 of GB-A-2172295). Thus, the elastic component of the complex dynamic moduli (G′) is higher than the loss moduli (G″) for all tested frequencies. At the same time, the material behaves like a pseudo-plastic body at low shear rates, i.e. it has a substantial viscosity which decreases with shear rate. This material is also characterized by a very long relaxation time. We strongly believe that this is a unique structure of solutions of HY obtained according to the present invention which makes it possible to obtain the above described jelly-type product with these special rheological properties. In other words, the chemical changes in HA occurring in the recovery process according to the present invention affect not only the structure and properties of HY but also the properties of products obtained therefrom. Accordingly, we have found that when HA obtained according to methods known in the art, namely by protein removal with chloroform extractions, was used as a starting material for cross-linking with divinyl sulfone, an insoluble material was obtained with rheological properties substantially worse than those obtainable used HY of the present invention.

It should be understood that many other modified materials can be obtained through additional modification of the product obtained according to the present invention, such as strongly cross-linked gels, insoluble films, coatings, etc.

The following examples illustrate preferred embodiments of the invention. Reference will be made to the accompanying drawing which is a graph showing viscosity vs. shear rate for the product described in Example 13.

## EXAMPLE 1

This example illustrates obtaining ultra-high molecular weight, sterile, pyrogen-free and non-inflammatory hylan.

Chicken combs were extensively washed with a 1% solution of cetylpyridinium chloride in 0.15 M aqueous sodium chloride, then with deionized water and then finally were kept in a 1% aqueous sodium hydroxide solution at room temperature for 15 minutes. Finally, the combs were washed with deionized water and frozen. The frozen combs were cut with a slicer to pieces about 1-2 mm in thickness. 1 kg of the thus prepared combs was mixed with a treatment solution containing 2.5 1 of isopropanol, 0.1 1 of chloroform, 0.07 1 of 37% formalin, and 0.05 1 of a 50% aqueous solution of sodium acetate in a glass container which was sterilized by autoclaving and depyrogenated by heat at 200 °C for 4 hours. All labware used in this example was treated the same way for sterilization and depyrogenation. The treatment of the combs in the above solution was carried out for 24 hours at a temperature of about 20 °C with slow stirring. The liquid was then separated from the combs by filtration through a Buchner funnel without any filtering aid. The treated combs were then washed with 2 1 of isopropanol, separated from the solvent as described above and put into a glass column with a stainless steel screen at the bottom. All the above described and the following procedures were carried out in a laminar flow hood to prevent bacterial contamination. A slow stream of nitrogen filtered through a 0.2 mm membrane was blown through the column for about 4 hours to dry the combs from the residual solvents. Then the combs were mixed with 2.5 1 of sterile, pyrogen-free water in a glass beaker and extracted at a temperature not higher than 16 °C for 24 hours. The pH in the extraction step was kept at 10.5 with the addition of aqueous sodium hydroxide when necessary. The combs were separated from the extract by filtration through a Buchner funnel. The extract was additionally filtered through a cellulosic type filter material ("Micro-media"® M70, Ertel Engineering Co.) sterilized by autoclaving. The hylan concentration in the filtrate was 0.45 mg/ml. 20 gm of sodium acetate were dissolved in 50 ml of water and added to 2 1 of the filtrate. A white fibrous precipitate formed when the filtrate was mixed with 4 1 of isopropanol with slow stirring. The fibers were separated from the liquid, washed with 1 1 of fresh isopropanol for 24 hours, then separated from the liquid and dried in a vacuum oven equipped with a cold trap at ambient temperature for 72 hours at a residual pressure not higher than 50 mtorrs.

The combs after the first extraction were extracted a second time in essentially the same manner with the exception that the time of the extraction was 96 hours. The second extract was filtered as described above. The hylan concentration in the filtrate was 0.83 mg/ml. The fibrous product was isolated from the second filtrate and dried as described above. The yield of the products in each extraction and the following properties were evaluated: hexosamine/hexuronic acid ratio, protein content, combined formaldehyde content, limiting viscosity number, sterility, pyrogenicity and rheological properties. The procedures used

were those as described above, i.e. in GB-A-2172295. In order to increase the sensitivity of the formaldehyde determination, that procedure was modified in the following manner: the fibrous product was dissolved in water to a concentration of about 1 wt. % and freeze-dried with formation of a foam-like, light material which was subsequently pressed into a very dense tablet. This tablet was additionally dried at 80 °C for 4 hours at a residual pressure of about 100 mtorrs and used as a sample in formaldehyde determination. The weight of the sample was about 2 gm which brought the sensitivity of the method to about 10 times higher as compared to the original method. The properties of the two products were as follows in Table I:

TABLE I

| Property | | Product of the 1st Extraction | Product of the 2nd Extraction |
|---|---|---|---|
| Yield, gm | | 0.89 | 1.60 |
| hexosamine/hexuronic acid molar ratio | | 1.02 | 0.97 |
| protein content wt. % of hylan polymer | | 0.35 | 0.22 |
| combined formaldehyde content wt. % of hylan polymer | | 0.0006 | 0.00025 |
| limiting viscosity number cc/gm (0.15 M NaCl, 25 °C) | | 5,600 | 6,700 |
| Rheology of 1 wt. % solution in 0.15 M aqueous solution chloride: | | | |
| Shear viscosity (Pa.s) at shear rate | $0.01 s^{-1}$<br>$100 s^{-1}$ | 1,330<br>1.31 | 1,780<br>1.23 |
| Shear viscosity at zero shear rate, Pa.s | | 1,882 | 3,155 |
| Dynamic storage moduli (Pa) at frequency | 0.05 Hz<br>5.00 Hz | 73.1<br>203 | 100.0<br>240.0 |
| Frequency at "cross-over point" Hz | | 0.0095 | 0.0063 |

The dependence of viscosity on shear rate in the low shear rate region is shown in the accompanying drawing.

To evaluate the "biological purity" of the products, 1 wt. % solutions in sterile, pyrogen-free 0.15 M aqueous sodium chloride were prepared. Sterility of the solutions was determined according to USP XXI standards and both solutions were found to be sterile. The pyrogen content was determined according to USP XXI by LAL (Limulus Amebocytae Lysate) Test and expressed in EU (endotoxin units) per ml. For both solutions it was less than 6EU/ml. In addition, inflammatory activity of the solutions was evaluated using the Owl Monkey Eye Test described in U.S. Patent 4,141,973 (Feb. 27, 1979) and the solutions were found to be essentially non-inflammatory.

Thus, ultra-high molecular weight sterile, pyrogen-free, and non-inflammatory hylan with considerably smaller amounts of combined formaldehyde as compared to GB-A-2172295 was obtained.

## EXAMPLE 2

This example illustrates obtaining high molecular weight hylan with increased yield.

Chicken combs were extensively washed with 1% aqueous cetylpyridinium chloride solution, then with deionized water and, finally, frozen. The frozen combs were sliced into pieces of 1-2 mm thickness. 500 gm of sliced combs were put into a mixture of 600 ml of isopropanol, 50 ml of 37 wt. % formalin and 25 gm of sodium acetate and kept in this mixture for 24 hours at a temperature not higher than 25 °C. Then the combs were separated from the liquid and left to dry in air for about 2 hours. The thus dried combs were mixed with 1.2 1 of 0.04 N aqueous solution of sodium hydroxide which was preliminarily cooled to about 4 °C. The pH of the mixture was about 12. The extraction was carried out for 5 days at a temperature about 4-6 °C. Then, the combs were separated from the extract and the latter was filtered through the cellulosic filter media M-70. The hylan concentration in the filtrate was 3.2 mg/ml. 10 gm of sodium acetate were dissolved in 1 1 of the filtrate and the solution obtained was poured slowly into 2 1 of acetone. White fibers

precipitated from the solution which were collected, washed with fresh acetone and dried in a vacuum oven equipped with a cold trap at ambient temperature for 72 hours at residual pressure about 50 mtorrs. 3.05 gm of a dry fibrous material were obtained. This material had limiting viscosity number 6200 cc/gm, protein content 0.55 wt. % and combined formaldehyde content of 0.00035 wt. % and was thus identified as hylan.

EXAMPLE 3

This example illustrates the effect of a sodium hydroxide solution concentration and time of the extraction on hylan properties.

The comb preparation and the treatment and after-treatment drying steps were carried out essentially as described in the previous example. The dried combs were put into 1.2 1 of 0.2 N aqueous sodium hydroxide solution precooled to 4 °C. The pH of the mixture was higher than 14. About one-half of the extract was taken out after 42 hours of extraction at 4 °C. The hylan concentration was 4.2 mg/ml. The extract was filtered and hylan was precipitated as described in the previous example. White fibers were obtained with limiting viscosity number in 0.15 M aqueous NaCl at 25 °C 3200 cc/gm and protein content 0.4/wt. %. The rest of the extract was separated from the combs after 140 hours of total extraction time at 4 °C and was treated as described above. 2.2 gm of white fibers were recovered from this portion of the extract with limiting viscosity number 2300 cc/gm and protein content 0.85 wt. %. The combined formaldehyde content in each of these two products was about 0.003 wt. %.

Thus, when the alkali concentration in the extraction step is relatively high though the extraction at low temperature leads to products with a substantially higher limiting viscosity number as compared to extraction at ambient temperature. The increase in time of the extraction results in decrease of limiting viscosity number of the product.

EXAMPLE 4

This example illustrates the use of a volatile base in the extraction step. The combs were prepared and treated as described in the previous example. The combs were extracted with 1.2 1 of 0.25 wt. % solution of ammonia in water at 25 °C for 96 hours. The extract was separated from the combs, filtered and precipitated as described in the previous example. The hylan concentration in the fibrous material obtained had limiting viscosity number 4500 cc/gm and protein content 0.55 wt. %.

EXAMPLE 5

This example illustrates the effect of the temperature during the extraction step on the properties of the final product.

500 gm of rooster combs were washed, sliced and treated as described in the previous examples. The combs were then divided into two portions. One portion was extracted with 600 ml of 0.2 N aqueous sodium hydroxide solution containing 0.2% of ammonia at 25 °C for 96 hours. The extract was filtered (the hylan concentration in the filtrate was 5.3 mg/ml) and precipitated as described above. The limiting viscosity number of the product was 1900 cc/gm and the protein content 1.7 wt. %. The other portion of the combs was mixed with 600 ml of the same solution precooled to 4 °C and the extraction was carried out at 4 °C for 96 hours. The hylan concentration in the filtrate was 4.5 mg/ml, the limiting viscosity number of the product was 2700 cc/gm and the protein content 0.52 wt. %.

Thus, a decrease of the temperature in the extraction steps is seen to result in a higher molecular weight and a lower protein content.

EXAMPLE 6

This example illustrates the extraction of treated combs with a mixture of alkalies at a lower concentration.

500 gm of combs were pre-washed, sliced and treated with the formaldehyde containing mixture essentially as described in Example 2. The combs were put into 1.2 1 of a precooled to 4 °C 0.1 M sodium hydroxide solution containing 0.2% of ammonia. The extraction was carried out at 4 °C for 96 hours.

The extract obtained was separated, filtered and the product was precipitated as described in the previous examples. The hylan concentration in the extract was 4.9 mg/ml. 4.5 gm of fibers were obtained. The limiting viscosity number of the product was 3900 cc/gm and the protein content 0.25 wt. %.

The combs were extracted a second time essentially as described above. The hylan concentration in the extract was 4.0 mg/ml. 3.4 gm of fibers were obtained with the limiting viscosity number 4400 cc/gm and the protein content 0.65 wt. %.

EXAMPLE 7

This example illustrates the use of a quaternary ammonium compound for recovery of hylan from the filtrate.

500 gm of combs were washed, frozen and sliced as described in the previous examples. The combs were treated in a mixture containing 600 ml of isopropanol, 50 ml of 37 wt. % formalin, and 25 gm of sodium acetate for 24 hours at 4°C. The treated combs were separated from the liquid, dried in air at ambient temperature and then extracted with 1.2 1 of 0.1 N NaOH to which 25 ml of 25% ammonia solution was added. The extraction was carried out at 4°C for 48 hours. The extract was separated from the combs and neutralized with concentrated hydrochloric acid. The hylan concentration in the extract was 2.86 mg/ml. 500 ml of the filtered extract were precipitated in the usual way described in the previous examples with the use of isopropanol as a precipitant. 1.35 gm of the product was obtained which had a limiting viscosity number of 3900 cc/gm and a protein content of 0.5 wt. % (Product A). The other 500 ml portion of the filtered extract was mixed with 275 ml of 1 wt. % aqueous solution of cetylpyridinium chloride (CPC). A resinous precipitate formed which was separated from the supernatant liquid, washed 2 times with 500 ml of water at 50°C, then once with 500 ml of water at 20°C and, finally, dissolved in 60 ml of 0.3 M aqueous sodium chloride. 0.7 gm of sodium acetate was dissolved in this solution which was then poured slowly into 60 ml of isopropanol. A fibrous white precipitate formed which was washed 2 times with 50 ml of 1:1 water-isopropanol mixture and finally, with 50 ml of isopropanol and then dried in vacuum. 1.41 gm of a product with the limiting viscosity number 4000 cc/gm and a protein content of 0.6 wt. % was obtained (Product B). The CPC content in the product was 25 ppm. The rheological properties of the two products of 0.5 wt. % solutions in 0.15 M saline were the following:

| Rheological Data | Product A | Product B |
|---|---|---|
| Shear viscosity (Pa.s) at shear rate 0.01 $s^{-1}$ | 117 | 118 |
| Shear viscosity at zero shear rate, Pa.s | 371 | 315 |
| Dynamic storage moduli (Pa) at frequency 0.05 Hz | 4.80 | 3.64 |
| Frequency at "cross-over point" Hz | 0.04 | 0.05 |

Thus, the use of CPC for recovery of hylan from the filtrate gave a product with essentially the same properties as compared to the product obtained through precipitation with isopropanol.

EXAMPLE 8

This example describes an experiment with radiolabeled formaldehyde.

A solution of radiolabeled $^{14}CH_2O$ in water with a specific activity of 57 mCi/mmol (New England Nuclear) was mixed in an amount corresponding to 2 mCi (0.104 ml) with 1.0 ml of 37 wt. % formalin. Specific activity of the mixture was 0.15 mCi/mmol. This formaldehyde solution was added to 12.5 ml of isopropanol containing 0.5 gm of sodium acetate and 10 gm of sliced rooster combs were put into the mixture in a stoppered flask. The treatment of the combs was carried out for 18 hours at ambient temperatures (about 20°C). The combs were separated from the liquid, washed two times with 10 ml of isopropanol and dried in air to one-half of their original weight. 25 ml of distilled water was added to the combs and extraction was allowed to proceed for 48 hours at 4°C. The first extract (pH 7.0) was separated from the combs, filtered through nylon fabric and then filtered through four layers of Whatman® #1 filter paper. The second extract from the combs was prepared and treated in essentially the same manner.

Sodium acetate was added to both filtrates to make 1 wt. % solution. White fibers were precipitated from both filtrates by pouring the filtrate into a double volume of isopropanol. Hylan fibers were collected, washed extensively (3 times) with isopropanol, dried in a vacuum oven at ambient temperature and then redissolved in water to give solutions with a hylan concentration of about 1 mg/ml. The hylan fibers were reprecipitated as described above, washed with isopropanol and dried in vacuum oven. The dried fibers were redissolved in distilled water to give solutions containing 0.98 and 1.65 mg/ml of hylan for the products of the first and the second extractions, respectively. The protein content in these solutions was 5.98 and 12.0 ug/ml, respectively. The solutions were dialyzed, first, against saline-phosphate buffer (pH 7.5) and then against citrate-phosphate buffer (pH 5.6) and the specific activities were found to be 1330 dpm/mg and 2515 dpm/mg of hylan for the first and the second extractions products, respectively, which corresponded to the content of combined non-dialyzable formaldehyde in hylan of 0.0128 wt. % in these two products. The specific activity when related to the protein content was 222 dpm/ug and 210 dpm/ug of proteins, respectively.

The two solutions were subjected to enzymatic digestion with testicular hyaluronidase which digests hyaluronan and chondroitin sulfate at enzyme concentration of 2 ug/ml for 48 hours at a temperature of 37 °C. Aliquots of the dialyzed solutions were removed and analyzed by gel electrophoresis and autoradiography. The remaining samples were dialyzed exhaustively against acetate-EDTA buffer (pH 6.0). The hylan concentration in the dialyzed enzyme digests was below detectable level (10 ug/ml) and the radioactivity level was 260 dpm/ml and 330 dpm/ml for the first and the second product, respectively, or when calculated on the original protein content 43 dpm/ug and 28 dpm/ug of proteins. Thus, in this experiment a substantial portion of the radioactivity became dialyzable after enzymatic degradation of the hyaluronan moiety of hylan macromolecules.

In order to determine the amount of the dialyzable radioactivity a portion of the dialyzable glycosaminoglycans from the experiment with the first extraction product was lyophilized. It was found that about 80% of the radioactivity (1070 dpm) was removed after drying the material.

Autoradiography of the hyaluronidase digested hylan samples revealed the presence of radiolabeled bands in protein gels with molecular weights of 14,000, 21,000, 22,000, 24,000 and 66,000, with the greatest incorporation seen in the 66,000 band (estimated by intensity of bands). The 66,000 band was found to be one of the major bands in electrophoretic pattern for the proteins found in hylan.

## EXAMPLE 9

This example illustrates the use of hylan solutions without precipitation of the fibers in cosmetic formulations.

The treatment of the combs, extraction and filtration were carried out essentially as described in Example 2. The hylan concentration in the filtrate was 3.05 mg/ml, the protein content 0.8 wt. % calculated on hylan concentration and limiting viscosity number 5700 cm/gm.

0.28 gm of dry poly(acrylic acid) (Carpobol 940® B.F. Goodrich) was dispersed in 50 ml of distilled water with stirring. Then 4 gm of glycerol, 4 ml of 5 wt. % aqueous poly(vinyl pyrrolidone) (molecular weight about 360,000, Aldrich Chemical Co.), and 10 ml of 1 wt. % aqueous solution of poly(ethylene oxide) (Polyox® Coagulant, Union Carbide) were added to the dispersion. 100 ml of the above hylan filtrate was slowly added to the mixture with stirring and the total volume of the mixture was brought to 200 ml with distilled water. A clear soft gel which could be used as a moisturizing hand gel was obtained. The final composition of the gel was the following; wt. %:

| | |
|---|---|
| Carbopol 940 | 0.14 |
| Poly(vinyl pyrrolidone) | 0.10 |
| Polyox Coagulant | 0.025 |
| hylan | 0.16 |
| glycerol | 2.00 |
| preservative | 0.30 |
| water | 97.275 |

EXAMPLE 10

This is another example illustrating the direct use of hylan filtrate for cosmetic formulation.

10 gm of stearic acid (Dar-Chem® 14, Darling & Co) were mixed with 50 ml of the hylan filtrate prepared as described in the previous example and 0.8 gm of Lanoxide® 52 (PEG-40 Stearate, Lanaetec Products, Inc.) was added to the mixture which was then warmed up with stirring to about 80 °C and cooled immediately to room temperature. The obtained mixture constitutes part A of the final formulation.

Part B was prepared by mixing together 10 ml of 1 wt. % dispersion of Carbopol 940 in water and 2.5 ml of 5 wt. % aqueous poly(vinyl pyrrolidone) solution and 1.4 ml of 4% aqueous sodium hydroxide solution.

Part C was prepared by mixing the following ingredients together (amounts given in gm):

| | |
|---|---|
| Petrolatum (Cheseborough-Ponds, Inc.) | 1.0 |
| Cetyl alcohol (Aldrich Chemical Co.) | 1.0 |
| Crodamol PMP (Croda, Inc.) | 0.5 |
| Volpo 3 (Croda, Inc.) | 0.25 |
| Coconut oil (Acme-Hardesty Co., Inc.) | 1.0 |
| PEG-350 (Aldrich Chemical Co.) | 0.3 |
| Propylene glycol (Aldrich Chemical Co.) | 1.0 |
| Silicone F-754 (SWS Silicones Co.) | 1.5 |
| Perfume | 0.25 |
| Phenonip (Nipa) | 0.3 |

All three parts were mixed together with vigorous stirring and the total volume was brought to 100 gm with water. A soft cream with a pearly look and a soft feel when applied to the skin was obtained.

## Claims

**1.** A method of obtaining a chemically modified hyaluronic acid preparation comprising:

(a) treating animal tissue containing hyaluronic acid with an aqueous treating mixture including an aldehyde to effect chemical modification of the hyaluronic acid contained in the tissue, in situ,

(b) removing excess treating mixture from the reaction mixture,

(c) extracting the chemically modified hyaluronic acid from the treated animal tissue with water at a temperature below 16 °C and an alkaline pH of at least 9.5 for at least 6 hours,

(d) separating the extract containing the chemically modified hyaluronic acid from the treated animal tissue, and

(e) recovering the chemically modified hyaluronic acid from the extract.

**2.** A method according to claim 1, wherein the temperature in step (c) is below 5 °C.

**3.** A method according to Claim 1 or Claim 2, wherein the pH in step (c) is from pH 9.5 to pH 14.

**4.** A method according to any preceding claim, wherein the alkaline in step (c) is achieved using an inorganic base, an organic base or mixtures thereof.

**5.** A method according to Claim 4, wherein there is used an inorganic base selected from sodium hydroxide, potassium hydroxide, ammonium hydroxide, sodium carbonate and potassium carbonate, or an organic based selected from triethylamine and triethanolamine.

**6.** A method according to any preceding claim, wherein the aldehyde used in step (a) is formaldehyde, glutaraldehyde or glyoxal.

**7.** A method according to any preceding claim, wherein the aqueous treating mixture used in step (a) includes a water-miscible solvent which does not react with the aldehyde.

**8.** A method according to Claim 7, wherein the solvent is a lower ketone, preferably acetone or methylethyl ketone, a lower alcohol, preferably ethanol or isopropanol, or an aprotic solvent, preferably

dimethyl formamide, dimethyl acetamide or dimethyl sulfoxide.

**9.** A method according to Claim 7 or Claim 8, wherein the aqueous treating mixture optionally includes an electrolyte, preferably sodium acetate, and a water insoluble organic solvent, preferably chloroform.

**10.** A method according to any one of Claims 7-9, wherein the weight ratio of water to water-miscible solvent in the treating mixture is 1-5:4-8.5 and the weight ratio of water to aldehyde is 1-5:0.02-1.

**11.** A method according to Claim 9, wherein the treating mixture comprises, in parts by weight:

| | |
|---|---|
| water | 10-50 |
| water-miscible solvent | 40-85 |
| aldehyde | 0.2-10 |
| water insoluble solvent | 0.5-10 |
| electrolyte | 0-20 |

**12.** A method according to any preceding claim, wherein the weight ratio of treating mixture to tissue to be treated in step (a) is at least 10:1, and the treatment is effected for from 4-24 hours.

**13.** A method according to any preceding claim, wherein the weight ratio of water to treated tissue in step (c) is 2-5:1 based on the weight of the treated tissue.

**14.** A method according to any preceding claim, wherein the animal tissue is rooster, chicken or hen combs and the combs are cut into slices of 1-3 mm thickness.

**15.** A method according to any preceding claim, wherein excess treating mixture is removed from the reaction mixture in step (b) by washing the treated tissue with a solvent or a solvent-water mixture.

**16.** A method according to Claim 15, wherein the solvent used for washing the treated tissue is the same solvent as is used in the treating mixture.

**17.** A method according to any preceding claim, wherein the treated tissue is dried to 25-50% of the treated weight before the extraction step (c).

**18.** A method according to Claim 17, wherein drying is effected in air or nitrogen.

**19.** A method according to any preceding claim, wherein recovery is effected in step (e) by precipitation with a quaternary ammonium compound, preferably cetyl pyridinium chloride.

**20.** A method according to any preceding claim, wherein the extract is separated from the animal tissue in step (d) by filtration, centrifugation or decantation.

**21.** A method according to Claim 20, wherein separation is effected by a two-step filtration procedure comprising a first, or gross, filtration through a wide mesh to remove the pieces of animal tissue and a second, or fine, filtration through a cellulosic material.

**22.** A method according to any one of Claims 1-16, wherein the chemically modified hyaluronic acid is recovered from the extract by precipitation with a solvent, followed by washing and drying the resulting precipitated chemically modified hyaluronic acid.

**23.** A method according to Claim 22, wherein the solvent used for the precipitation is acetone, ethanol or isopropanol.

**24.** A method according to Claim 23, wherein a mineral acid, preferably HCl, $H_2SO_4$ or $H_3PO_4$, or an electrolyte, preferably sodium acetate or NaCl, is added during the precipitation step.

**25.** A method according to any preceding claim, wherein the chemically modified hyaluronic acid is removed from the extract by lyophilization.

**26.** A method according to any preceding claim, and further comprising, washing said animal tissue in an uncut state with an inorganic alkali in an amount and concentration sufficient to create a pH of more than 12 for at least 5 minutes, prior to step (a).

**27.** A method according to Claim 26, wherein the inorganic alkali is NaOH at a concentration of 0.05 M to 4 M.

**28.** A method according to any preceding claim and further comprising neutralizing the excess alkali used in the extracting step (c) by adding an acid to thereby render the preparation suitable for use in products requiring a substantially neutral pH.

**29.** A method according to Claim 28, wherein the acid is an inorganic acid, preferably HCl, $H_2SO_4$ or $H_3PO_4$; an organic acid, preferably acetic, citric, stearic, palmitic, lauric or p-aminobenzoic acid; or a polymeric acid, preferably polyacrylic acid, polymethacrylic acid polyvinylsulfuric acid or polystyrene sulfuric acid.

**30.** A method according to any preceding claim, wherein there is recovered in step (e) a chemically modified hyaluronic acid preparation characterized by the presence of from 0.0002 to 0.005% by weight of aldehyde cross-linking groups covalently bonded to the hyaluronic acid polymer chains.

**31.** A method according to any preceding claim, wherein there is recovered in step (e) a chemically modified hyaluronic acid which is sterile, non-inflammatory and pyrogen-free.

**32.** A method of cross-linking a chemically modified hyaluronic acid obtained by a method according to any preceding claim, which comprises subjecting said chemically modified hyaluronic acid to a cross-linking reaction with divinyl sulfone under alkaline conditions at room temperature for about one hour.

**33.** A cosmetic formulation, comprising a prepartion obtained according to a method according to Claim 28.

**Patentansprüche**

**1.** Verfahren zur Herstellung eines chemisch modifizierten Hyaluronsäurepräparats, das folgende Schritte umfaßt:

a) Behandlung tierischer Gewebe, die Hyaluronsäure enthalten, mit einem wäßrigen, ein Aldehyd enthaltenden Behandlungsgemisch zwecks chemischer Modifizierung der in dem Gewebe enthaltenen Hyaluronsäure in situ,

b) Abtrennung des überschüssigen Behandlungsgemischs vom Reaktionsgemisch,

c) mindestens sechsstündige Extraktion der chemisch modifizierten Hyaluronsäure aus dem behandelten tierischen Gewebe mit Wasser bei einer Temperatur unter 16° C und einem alkalischen pH-Wert von mindestens 9,5,

d) Abtrennung des die chemisch modifizierte Hyaluronsäure enthaltenden Extrakts von dem behandelten tierischen Gewebe und

e) Rückgewinnung der chemisch modifizierten Hyaluronsäure aus dem Extrakt.

**2.** Verfahren nach Anspruch 1, dadurch gekennzeichnet, daß die in Schritt (c) verwendete Temperatur weniger als 5° C beträgt.

**3.** Verfahren nach Anspruch 1 oder Anspruch 2, dadurch gekennzeichnet, daß der in Schritt (c) genutzte pH-Wert zwischen 9,5 und 14 liegt.

**4.** Verfahren nach einem der vorstehenden Ansprüche, dadurch gekennzeichnet, daß der alkalische Zustand in Schritt (c) durch Verwendung
einer anorganischen Base, einer organischen Base oder deren Gemischen erzielt wird.

**5.** Verfahren nach Anspruch 4, dadurch gekennzeichnet, daß eine anorganische, unter Natriumhydroxid, Kaliumhydroxid, Ammoniumhydroxid, Natriumkarbonat und Kaliumkarbonat ausgewählte Base oder eine organische, unter Triethylamin und Triethanolamin ausgewählte Base verwendet wird.

**6.** Verfahren nach einem der vorstehenden Ansprüche, dadurch gekennzeichnet, daß das in Schritt (a) verwendete Aldehyd Formaldehyd, Glutaraldehyd oder Glyoxal ist.

**7.** Verfahren nach einem dar vorstehenden Ansprüche, dadurch gekennzeichnet, daß das in Schritt (a) verwendete wäßrige Behandlungsgemisch ein mit Wasser mischbares Lösemittel enthält, das nicht mit dem Aldehyd reagiert.

**8.** Verfahren nach Anspruch 7, dadurch gekennzeichnet, daß das Lösemittel ein niederes Keton, vorzugsweise Aceton oder Methylethylketon, ein niederer Alkohol, vorzugsweise Ethanol oder Isopropanol, oder ein aprotisches Lösemittel, vorzugsweise Dimethylformamid, Dimethylacetamid oder Dimethylsulfoxid, ist.

**9.** Verfahren nach Anspruch 7 oder Anspruch 8, dadurch gekennzeichnet, daß das wäßrige Behandlungsgemisch wahlweise einen Elektrolyten, vorzugsweise Natriumacetat, und ein wasserunlösliches organisches Lösemittel, vorzugsweise Chloroform, enthält.

**10.** Verfahren nach einem der Ansprüche 7-9, dadurch gekennzeichnet, daß das Masseverhältnis von Wasser und dem mit Wasser mischbaren Lösemittel im Behandlungsgemisch 1-5 : 4-8,5 und das Masseverhältnis von Wasser zu Aldehyd 1-5 : 0,02-1 beträgt.

**11.** Verfahren nach Anspruch 9, dadurch gekennzeichnet, daß das Behandlungsgemisch enthält:

| | in Masseteilen |
|---|---|
| Wasser | 10-50 |
| mit Wasser mischbares Lösemittel | 40-85 |
| Aldehyd | 0,2-10 |
| wasserunlösliches Lösemittel | 0,5-10 |
| Elektrolyt | 0-20 |

**12.** Verfahren nach einem der vorstehenden Ansprüche, dadurch gekennzeichnet, daß das Masseverhältnis des Behandlungsgemischs zum Gewebe, das in Schritt (a) zu behandeln ist, mindestens 10 : 1 beträgt und die Behandlung 4 bis 24 Stunden in Anspruch nimmt.

**13.** Verfahren nach einem der vorstehenden Ansprüche, dadurch gekennzeichnet, daß das Masseverhältnis von Wasser zum in Schritt (c) behandelten Gewebe 2-5 : 1, bezogen auf das Gewicht des behandelten Gewebes, beträgt.

**14.** Verfahren nach einem der vorstehenden Ansprüche, dadurch gekennzeichnet, daß Kämme von Hähnen, Hähnchen oder Hennen als tierisches Gewebe eingesetzt und die Kämme in 1-3 mm dicke Scheiben geschnitten werden.

**15.** Verfahren nach einem der vorstehenden Ansprüche, dadurch gekennzeichnet, daß in Schritt (b) überschüssiges Behandlungsgemisch durch Waschen des behandelten Gewebes mit einem Lösemittel oder einem Lösemittel-Wasser-Gemisch aus dem Reaktionsgemisch entfernt wird.

**16.** Verfahren nach Anspruch 15, dadurch gekennzeichnet, daß zum Waschen des behandelten Gewebes das gleiche Lösemittel wie im Behandlungsgemisch verwendet wird.

**17.** Verfahren nach einem der vorstehenden Ansprüche, dadurch gekennzeichnet, daß das behandelte Gewebe vor dem Extraktionsschritt (c) auf 25-50 % des Gewichts nach der Behandlung heruntergetrocknet wird.

**18.** Verfahren nach Anspruch 17, dadurch gekennzeichnet, daß die Trocknung in Luft oder Stickstoff erfolgt.

**19.** Verfahren nach einem der vorstehenden Ansprüche, dadurch gekennzeichnet, daß in Schritt (e) die Rückgewinnung durch Fällen mit einer quartären Ammoniumverbindung, vorzugsweise Cetylpyridiniumchlorid, erfolgt.

**20.** Verfahren nach einem der vorstehenden Ansprüche, dadurch gekennzeichnet, daß in Schritt (d) der Extrakt durch Filtration, Zentrifugation oder Dekantation von dem tierischen Gewebe abgetrennt wird.

**21.** Verfahren nach Anspruch 20, dadurch gekennzeichnet, daß die Abtrennung durch ein zweistufiges Filtrationsverfahren erfolgt, das eine erste oder Grobfiltration durch ein weitmaschiges Filter zwecks Entfernung der Stücke tierischen Gewebes, und eine zweite oder Feinfiltration durch ein Cellulosematerial umfaßt.

**22.** Verfahren nach einem der Ansprüche 1-16, dadurch gekennzeichnet, daß die chemisch modifizierte Hyaluronsäure aus dem Extrakt durch Fällen mit einem Lösemittel mit anschließendem Waschen und Trocknen der erhaltenen ausgefällten chemisch modifizierten Hyaluronsäure erfolgt.

**23.** Verfahren nach Anspruch 22, dadurch gekennzeichnet, daß das zum Fällen genutzte Lösemittel Aceton, Ethanol oder Isopropanol ist.

**24.** Verfahren nach Anspruch 23, dadurch gekennzeichnet, daß während des Fällungsschrittes eine Mineralsäure, vorzugsweise HCl, $H_2SO_4$ oder $H_3PO_4$, oder ein Elektrolyt, vorzugsweise Natriumacetat oder NaCl, zugesetzt wird.

**25.** Verfahren nach einem der vorstehenden Ansprüche, dadurch gekennzeichnet, daß die chemisch modifizierte Hyaluronsäure aus dem Extrakt durch Gefriertrocknung entfernt wird.

**26.** Verfahren nach einem der vorstehenden Ansprüche, dadurch gekennzeichnet, daß es darüber hinaus vor Schritt (a) ein mindestens fünfminütiges Waschen des unzerkleinerten tierischen Gewebes mit einem anorganischen Alkali in einer Menge und Konzentration umfaßt, die genügen, um einen pH-Wert von über 12 zu erzielen.

**27.** Verfahren nach Anspruch 26, dadurch gekennzeichnet, daß das anorganische Alkali NaOH in einer Konzentration von 0,05 M bis 4 M ist.

**28.** Verfahren nach einem der vorstehenden Ansprüche, dadurch gekennzeichnet, daß es darüber hinaus im verwendeten Extraktionsschritt (c) die Neutralisierung des überschüssigen Alkali durch Zugabe einer Säure umfaßt, um das Präparat auf diese Weise geeignet zu machen für den Einsatz in Produkten, die einen im wesentlichen neutralen pH-Wert erfordern.

**29.** Verfahren nach Anspruch 28, dadurch gekennzeichnet, daß die Säure eine anorganische Säure, vorzugsweise HCl, $H_2SO_4$ oder $H_3PO_4$; eine organische Säure, vorzugsweise Essig-, Zitronen-, Stearin-, Palmitin-, Laurinsäure oder p-Aminobenzoesäure; oder eine polymere Säure, vorzugsweise Polyacrylsäure, Polymethacrylsäure, Polyvinylschwefelsäure oder Polystyrenschwefelsäure, ist.

**30.** Verfahren nach einem der vorstehenden Ansprüche, dadurch gekennzeichnet, daß in Schritt (e) ein chemisch modifiziertes Hyaluronsäurepräparat rückgewonnen wird, das gekennzeichnet ist durch die Gegenwart von 0,0002 bis 0,005 Gew.-% Aldehyd-Vemetzungsgruppen, die an die Polymerketten der Hyaluronsäure kovalent gebunden sind.

**31.** Verfahren nach einem der vorstehenden Ansprüche, dadurch gekennzeichnet, daß in Schritt (e) eine chemisch modifizierte Hyaluronsäure rückgewonnen wird, die steril, antiphlogistisch und pyrogenfrei ist.

**32.** Verfahren zur Vernetzung einer chemisch modifizierten Hyaluronsäure, die durch ein Verfahren nach einem der vorstehenden Ansprüche gewonnen wurde, dadurch gekennzeichnet, daß die chemisch modifizierte Hyaluronsäure unter alkalischen Bedingungen einer etwa einstündigen Vernetzungsreaktion mit Divinylsulfon bei Raumtemperatur unterworfen wird.

**33.** Eine kosmetische Zubereitung, dadurch gekennzeichnet, daß sie ein Präparat enthält, das nach einem Verfahren nach Anspruch 28 hergestellt wurde.

**Revendications**

**1.** Procédé d'obtention d'une préparation d'acide hyaluronique modifié chimiquement, comprenant les étapes consistant à:
(a) traiter un tissu animal contenant de l'acide hyaluronique avec un mélange de traitement aqueux contenant un aldéhyde pour effectuer une modification chimique in situ de l'acide hyaluronique contenu dans le tissu,
(b) éliminer du mélange réactionnel l'excès de mélange de traitement,
(c) extraire l'acide hyaluronique modifié chimiquement du tissu animal traité avec de l'eau présentant une température inférieure à 16 °C et un pH alcalin d'au moins 9,5 pendant au moins 6 heures,
(d) séparer l'extrait contenant l'acide hyaluronique modifié chimiquement du tissu animal traité, et
(e) récupérer l'acide hyaluronique modifié chimiquement à partir de l'extrait.

**2.** Procédé selon la revendication 1, dans lequel la température dans l'étape (c) est inférieure à 5 °C.

**3.** Procédé selon la revendication 1 ou la revendication 2, dans lequel le pH dans l'étape (c) est de pH 9,5 à pH 14.

**4.** Procédé selon l'une quelconque des revendications précédentes, dans lequel le pH alcalin, dans l'étape (c), est obtenu à l'aide d'une base minérale, d'une base organique ou de mélanges de celles-ci.

**5.** Procédé selon la revendication 4, dans lequel on utilise une base minérale choisie parmi l'hydroxyde de sodium, l'hydroxyde de potassium, l'hydroxyde d'ammonium, le carbonate de sodium et le carbonate de potassium, ou une base organique choisie parmi la triéthylamine et la triéthanolamine.

**6.** Procédé selon l'une quelconque des revendications précédentes, dans lequel l'aldéhyde utilisé dans l'étape (a) est le formaldéhyde, le glutaraldéhyde ou le glyoxal.

**7.** Procédé selon l'une quelconque des revendications précédentes, dans lequel le mélange de traitement aqueux utilisé dans l'étape (a) contient un solvant miscible avec l'eau qui ne réagit pas avec l'aldéhyde.

**8.** Procédé selon la revendication 7, dans lequel le solvant est une cétone inférieure, de préférence l'acétone ou la méthyléthylcétone, un alcool inférieur, de préférence l'éthanol ou l'isopropanol, ou un solvant aprotique, de préférence le diméthylformamide, le diméthylacétamide ou le diméthylsulfoxyde.

**9.** Procédé selon la revendication 7 ou la revendication 8, dans lequel le mélange de traitement aqueux contient éventuellement un électrolyte, de préférence l'acétate de sodium, et un solvant organique insoluble dans l'eau, de préférence le chloroforme.

**10.** Procédé selon l'une quelconque des revendications 7-9, dans lequel le rapport pondéral de l'eau au solvant miscible avec l'eau, dans le mélange de traitement, est de 1-5:4-8,5 et le rapport pondéral de l'eau à l'aldéhyde est de 1-5:0,02-1.

**11.** Procédé selon la revendication 9, dans lequel le mélange de traitement comprend, en parties en poids:

| | |
|---|---|
| eau | 10-50 |
| solvant miscible avec l'eau | 40-85 |
| aldéhyde | 0,2-10 |
| solvant insoluble dans l'eau | 0,5-10 |
| électrolyte | 0-20 |

**12.** Procédé selon l'une quelconque des revendications précédentes, dans lequel le rapport pondéral du mélange de traitement au tissu à traiter dans l'étape (a) est d'au moins 10:1, et le traitement est réalisé

pendant de 4 à 24 heures.

**13.** Procédé selon l'une quelconque des revendications précédentes, dans lequel le rapport pondéral de l'eau au tissu traité dans l'étape (c) est de 2-5:1, par rapport au poids du tissu traité.

**14.** Procédé selon l'une quelconque des revendications précédentes, dans lequel le tissu animal est une crête de coq, de poulet ou de poule et la crête est découpée en tranches de 1-3 mm d'épaisseur.

**15.** Procédé selon l'une quelconque des revendications précédentes, dans lequel l'excès de mélange de traitement est éliminé du mélange réactionnel dans l'étape (b) par lavage du tissu traité avec un solvant ou un mélange de solvant et d'eau.

**16.** Procédé selon la revendication 15, dans lequel le solvant utilisé pour laver le tissu traité est le même solvant que celui utilisé dans le mélange de traitement.

**17.** Procédé selon l'une quelconque des revendications précédentes, dans lequel le tissu traité est séché à 25-50% du poids traité avant l'étape d'extraction (c).

**18.** Procédé selon la revendication 17, dans lequel le séchage est effectué dans de l'air ou dans de l'azote.

**19.** Procédé selon l'une quelconque des revendications précédentes, dans lequel la récupération est réalisée, dans l'étape (e), par précipitation avec un composé d'ammonium quaternaire, de préférence le chlorure de cétylpyridinium.

**20.** Procédé selon l'une quelconque des revendications précédentes, dans lequel l'extrait est séparé du tissu animal, dans l'étape (d), par filtration, centrifugation ou décantation.

**21.** Procédé selon la revendication 20, dans lequel la séparation est effectuée par une filtration à deux étapes comprenant une première filtration, ou filtration grossière, à travers de grosses mailles, pour éliminer les morceaux de tissu animal, et une seconde filtration, ou filtration fine, à travers une matière cellulosique.

**22.** Procédé selon l'une quelconque des revendications 1-16, dans lequel l'acide hyaluronique modifié chimiquement est récupéré à partir de l'extrait par précipitation avec un solvant, suivie d'un lavage et d'un séchage de l'acide hyaluronique modifié chimiquement précipité résultant.

**23.** Procédé selon la revendication 22, dans lequel le solvant utilisé pour la précipitation est l'acétone, l'éthanol ou l'isopropanol.

**24.** Procédé selon la revendication 23, dans lequel on ajoute, au cours de l'étape de précipitation, un acide minéral, de préférence HCl, $H_2SO_4$ ou $H_3PO_4$, ou un électrolyte, de préférence l'acétate de sodium ou NaCl.

**25.** Procédé selon l'une quelconque des revendications précédentes, dans lequel l'acide hyaluronique modifié chimiquement est séparé de l'extrait par lyophilisation.

**26.** Procédé selon l'une quelconque des revendications précédentes, comprenant, en outre, le lavage dudit tissu animal à l'état non découpé avec une base minérale, en une quantité et une concentration suffisantes pour créer un pH supérieur à 12 pendant au moins 5 minutes, avant l'étape (a).

**27.** Procédé selon la revendication 26, dans lequel la base minérale est NaOH, en concentration de 0,05M à 4M.

**28.** Procédé selon l'une quelconque des revendications précédentes, comprenant, en outre, la neutralisation de l'excès de base utilisée dans l'étape d'extraction (c) grâce à l'addition d'un acide, pour rendre la préparation utilisable dans des produits nécessitant un pH pratiquement neutre.

**29.** Procédé selon la revendication 28, dans lequel l'acide est un acide minéral, de préférence HCl, $H_2SO_4$ ou $H_3PO_4$; un acide organique, de préférence l'acide acétique, citrique, stéarique, palmitique, laurique ou p-aminobenzoïque; ou un acide polymère, de préférence un poly(acide acrylique), un poly(acide méthacrylique), un poly(acide vinylsulfurique) ou un poly(acide styrène-sulfonique).

**30.** Procédé selon l'une quelconque des revendications précédentes, dans lequel on récupère, dans l'étape (e), une préparation d'acide hyaluronique modifié chimiquement caractérisée par la présence de 0,0002 à 0,005% en poids de groupes de réticulation aldéhyde liés par covalence aux chaînes polymères d'acide hyaluronique.

**31.** Procédé selon l'une quelconque des revendications précédentes, dans lequel on récupère, dans l'étape (e), un acide hyaluronique modifié chimiquement qui est stérile, non inflammatoire et apyrogène.

**32.** Procédé de réticulation d'un acide hyaluronique modifié chimiquement obtenu par un procédé selon l'une quelconque des revendicatlons précédentes, qui comprend les étapes consistant à soumettre ledit acide hyaluronique modifié chimiquement à une réaction de réticulation avec de la divinylsulfone, dans des conditions alcalines, à la température ambiante, pendant environ une heure.

**33.** Formulation cosmétique, comprenant une préparation obtenue par un procédé selon la revendication 28.

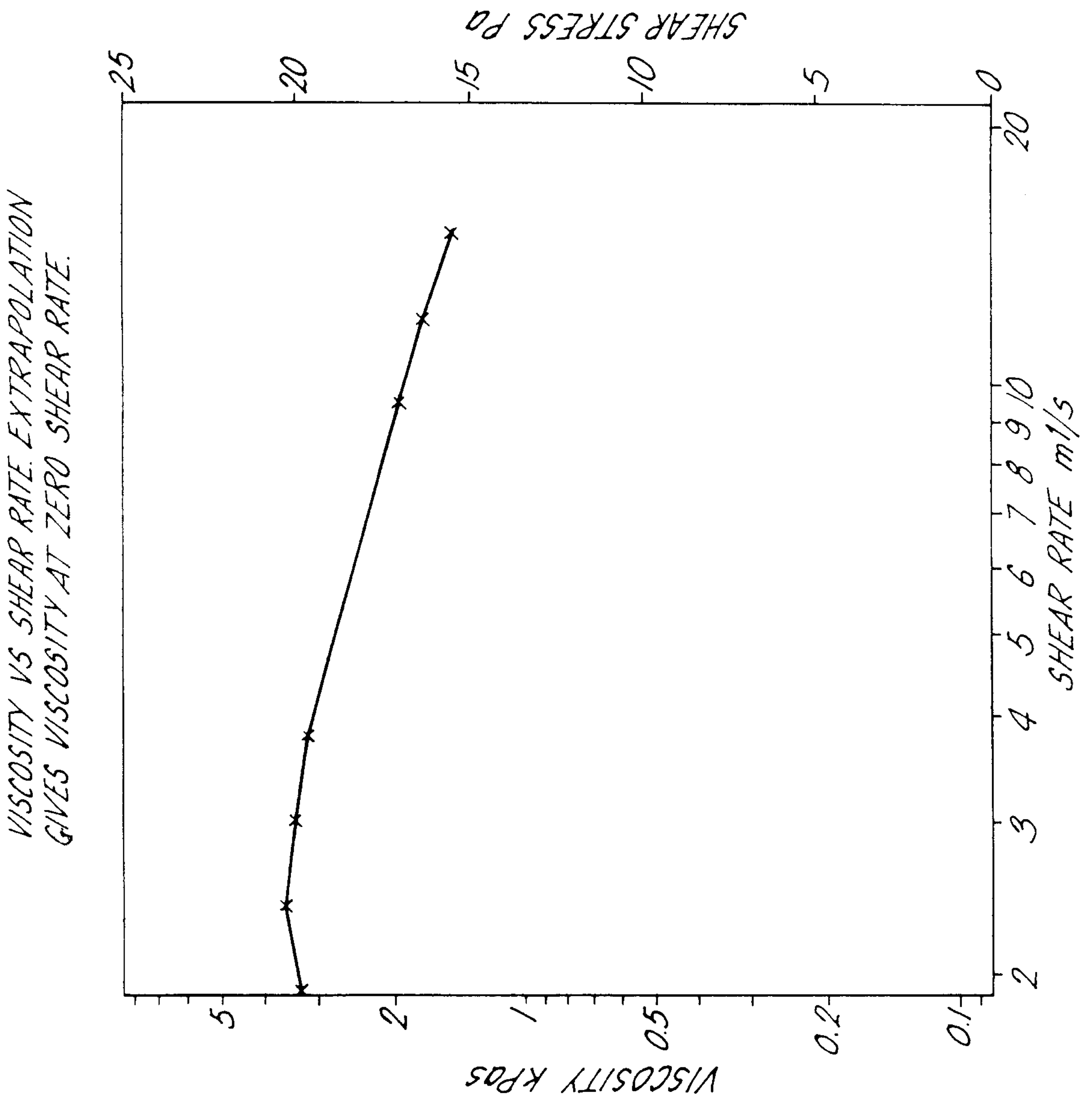
VISCOSITY VS SHEAR RATE. EXTRAPOLATION
GIVES VISCOSITY AT ZERO SHEAR RATE.
SHEAR STRESS Pa
25
20
15
10
5
0
SHEAR RATE m1/s
2
3
4
5
6
7
8
9
10
20
VISCOSITY kPas
5
2
1
0.5
0.2
0.1